# EUROPEAN PATENT SPECIFICATION

(11) **EP 1 739 161 B1**
(45) Date of publication and mention of the grant of the patent: **01.04.2009**
(21) Application number: 05013259.6
(22) Date of filing: 20.06.2005
(51) Int. Cl.: C11D 1/06

(54) **Foam-enhancing agent**
Schaumfördernde Zusammensetzung
composition promouvant l'équme

(43) Date of publication of application: 03.01.2007
(73) Proprietor: KAO CHEMICALS GmbH, 46446 Emmerich (DE)
(72) Inventor: Denzer, Horst, D-46446 Emmerich (DE); Michaelsen Marco, D-46446 Emmerich (DE); Meijer, Hamke, D-46446 Emmerich (DE); Abe, Hiroshi, 08210 Barbera del Valles (Barcelona) (ES)
(74) Representative: HOFFMANN EITLE

(56) References cited:
- EP-A- 0 344 442
- EP-A- 0 357 561
- WO-A-00/29530
- US-A- 6 143 307

## Description

### Technical field

The invention relates to liquid concentrated aqueous solutions of alkyl ether carboxylates derived from alkanols having 6 to 22 carbon atoms and having an average ethoxylation degree from 2.0 to 4.5, and glycerol, and to the use of these concentrated aqueous solutions as foam-enhancing agent or foam booster for anionic, cationic, non-ionic and/or amphoteric surfactants.

### Prior Art

In a number of surfactant applications, consumers are looking for a high foaming capacity. For example, a shampoo that does not produce enough creamy, stable foam during shampooing has no chance of success on the market. The same applies to manual dishwashing detergents, even though a direct connection between the foaming capacity and the cleaning performance cannot be established at all in many cases.

Hence, besides performance requirements, such as cleaning performance and dermatological compatibility, the foaming behaviour is a further important product feature. However, not all surfactant mixtures which perform satisfactorily and are economical in use show the required foaming behaviour.

The main foaming characteristics of the surfactant formulations that determine their application in areas like personal and house-hold care, washing, food industry, fire fighting, mineral flotation, and many others are: foaming ability, foam stability (foam remaining after certain period at rest), foam quantity (associated with good cleaning effect), creaminess of the foam (associated with conditioning effect), foam density, texture of the foam and foam speed (foam produced after a very short period of time). Insufficient foaming performance can thus for example materialize in an insufficient foaming power or in an insufficient foam stability. In the one case, the basic foam has sufficient height, but it collapses rapidly. In the other case, the exact opposite occurs, i.e. although the initial foaming height is comparably low, the foam remains stable for a long time. Moreover, it is desirable that the foam is obtained quickly upon use (e.g. after a few seconds). Furthermore, the foam must tolerate hard water and the presence of oil.

The number of surfactant combinations that meet this complex requirement profile tends to be small which explains why the same formulations are always found on the market.

One way of overcoming this problem would be to provide surfactant formulations with additives, so-called foam boosters or foam-enhancing agents, which favourably influence the foam properties of the mixtures. Traditionally, betaines, like cocamidopropyl betaine, have been used as foam boosters or foam-enhancing agents.

It is also known that compositions comprising alkyl ether carboxylates can be used as foam boosters.

The International patent application WO-A-2004104151 describes aqueous composition comprising:
a) alkyl ether carboxylates having an average ethoxylation degree in the range of 2-10; and
b) ethoxylated glycerine, having an ethoxylation degree in the range of 1-15 per mol of glycerine the weight ratio of component (a) to component (b) being in the range of 4:1 to 1:10.

Furthermore, in the Euro Cosmetics Magazine (ISSN 0944-8942), Volume No. 12, Special Issue 2004, in the section Cosmetic Raw Material, it is described a foam booster, Akypo ^{®} Foam LG37, which is a mixture of Sodium Laureth-4 Carboxylate and Glycereth-7 having a 22 wt. % active matter.

Alkyl ether carboxylates have the disadvantage that they are water soluble to a limited degree only, while retaining the liquid properties of the solution. The requirement generally imposed on these solutions is that they should be pumpable and homogeneous at room temperature. The solutions do not need to be completely clear; some turbidity does not detract from the homogeneity. At higher concentrations gelling generally occurs. In general, products can hardly, if at all, be processed if the viscosity exceeds 120,000 mPa.s. As a rule, the concentration of the aqueous solutions will therefore not exceed 25 wt.%, which has drawbacks with regard to transport and storage capacity. At such low concentrations, it is generally also necessary to preserve the products from microbiological contamination.

There are several patent and patent applications related to processes for obtaining high concentrated aqueous solutions of alkyl ether carboxylates.

EP-A-0344442 describes a process for the preparation of concentrated pumpable alkyl ether carboxylates wherein alkyl ether carboxylic acids of the structure

R-O-(A-O)ₙ-CH₂-COOH,

wherein R is a C₈-C₂₄ alkyl group or a C₉-C₂₄ alkylaryl group; A is an C₂-C₅ alkylene group and n=1-30, preferably 5-20; are neutralized, and an alcohol is added before, during or after the neutralization. The carboxymethylation degree of the alkyl ether carboxylic acids is higher than 90 mol.%. It is specified that the alcohol could be present from 0.5 to 15 wt.% being a C₁-C₆ monohydric to trihydric alcohol. Examples of said alcohols are methanol, ethanol, n-propanol, i-propanol, n-butanol, i-butanol, t-butanol, pentanol, ethylenglycol, propylenglycol, neopentylglycol, 1,4-butandiol, 1,6-hexandiol or glycerine. However, preferred alcohols are ethanol and i-propanol. Said alcohols have an undesirable low flash point and spread an unpleasant odour. Furthermore, EP-A-0344442 fails to address the foaming properties of these concentrated pumpable alkyl ether carboxylates.

EP-A-0357561 describes stable, concentrated aqueous solutions of alkyl ether carboxylates which are obtained by using a combination of solubilisers consisting of at least two components of the same or different category of the groups (A) monoethers of glycol or diglycol and (B) alkylenediol, where one of the components may have been replaced by a C₃-C₆ alcohol or such an alcohol is present as a further component. In the examples, alkyl ether carboxylates having a carboxymethylation degree of 80 mol.%. are used. Furthermore, EP-A-0357561 fails to address the foaming properties of these concentrated aqueous solutions of alkyl ether carboxylates.

The international patent application WO-A-0029530 describes a method for producing low-viscosity aqueous detergent preparations. According to said method, ethylene carboxylic acids or their salts of the formula R¹(OCH₂CH₂)ₙOCH₂COOX are added to aqueous solutions of viscosity-reducing additives, R¹ representing a linear and/or branched alkyl-, alkenyl- and/or alkylphenyl radical with 6 to 22 carbon atoms, X representing hydrogen, an alkaline metal, ammonium or alkylammonium and n representing numbers from 1 to 14. Among suitable viscosity-reducing additives, propoxylated glycerine is disclosed. However, WO-A-0029530 fails to address the foaming properties of these low-viscosity aqueous detergent preparations.

Finally, reference is made to EP-A-0580263. This patent relates to the provision of pumpable, highly concentrated (having a solid content of at least 45 wt.-%) liquid aqueous solutions of salts of alkyl ether carboxylic acids derived from alkanols having at least 10 carbon atoms, additionally containing ethoxylated, or ethoxylated and carboxymethylated, products or mixtures thereof, derived from polyhydric alcohols, the degree of ethoxylation of those products being at least 0.5. The ethoxylated, and/or ethoxylated and carboxymethylated products are contained in an amount of at least 1 %, in particular 2 to 25 %, relative to the total amount of solids of ether carboxylic acid and ethoxylated and/or ethoxylated and carboxymethylated products. Again, EP-A-0580263 fails to address the foaming properties of these surfactant mixtures.

U.S. 6,143,307 describes a composition containing laureth-11 carboxylic acid and glycerol.

### Summary of the invention

In view of this prior art, the complex problem addressed by the present invention is to provide liquid concentrated aqueous solutions of alkyl ether carboxylates which are pumpable and homogeneous without using strong agitation mediums (like homogenizers) and, at the same time, can be used as foam-enhancing agents that improve different foaming characteristics such as the basic foam, the foam speed, the foam stability, etc., of a large number of surfactants, even in the presence of hard water and oil, which are the most usual conditions for consumers.

In order to solve this problem, the present invention provides an aqueous composition comprising:
(a) one or more alkyl ether carboxylates of the following formula I

   R-O-(CH₂CH₂O)ₙ-CH₂COO⁻M⁺ Formula I

   wherein
   R is an alkyl residue having 6 to 22 carbon atoms;
   n has a value in the range of 2.0 to 4.5;
   and M+ is an appropriate cation, selected from the group consisting of an alkali metal, an alkaline earth metal, ammonium, an alkylammonium, an alkanolammonium or a glucammonium; and
(b) glycerol
the composition containing at most 55 wt.% water, based on the total weight of the composition.

The present invention also provides a process for the preparation of aqueous compositions of the current invention.

The present invention also provides a method for improving foam properties of anionic, cationic, non-ionic and/or amphoteric surfactants or mixtures thereof, which comprises combining an aqueous composition of the current invention with at least one surfactant.

The present invention also provides the use of aqueous compositions of the current invention as foam-enhancing agent or foam booster for anionic, cationic, non-ionic and/or amphoteric surfactants or mixtures thereof.

### Detailed description of the invention

Preferably, the aqueous compositions of the present invention comprise:
(a) one or more alkyl ether carboxylates of the following formula I

   R-O-(CH₂CH₂O)ₙ-CH₂COO⁻ M⁺ Formula I

   wherein
   R is an alkyl residue having 6 to 22 carbon atoms;
   n has a value in the range of 2.0 to 4.5;
   and M+ is an appropriate cation, selected from the group consisting of an alkali metal, an alkaline earth metal, ammonium, an alkylammonium, an alkanolammonium or a glucammonium;
(b) glycerol
   the composition containing at most 45 wt.%, more preferably at most 40 wt.% water, based on the total weight of the composition.

Particularly preferred are compounds of the above formula (I) wherein R is an alkyl residue having 12 to 14 carbon atoms, particularly one having 12 carbon atoms; n (representing the average ethoxylation degree) has a value in the range of 2.5 to 4.5 and M⁺ is an alkali metal cation such as sodium or potassium. Particularly preferred are compounds of the above formula (I) wherein R is an alkyl residue having 12 to 14 carbon atoms and n is 3.0 or 4.0.

Alkyl ether carboxylates are usually obtained by alkoxylation and subsequent carboxymethylation of fatty alcohols.

The process is divided into two steps. The first one is the alkoxylation of alcohols under standard conditions known by the skilled in the art. For instance, the polyoxyethylene group is obtained by addition of ethylene oxide to fatty alcohols, mostly with an alkaline catalyst such as NaOH, KOH or NaOCH₃, giving a broad polyoxyethylene oxide distribution (broad ethoxylation degree). For special applications the ethoxylation can be catalyzed by Lewis acids or by using metallic Na or NaH to achieve a narrow range distribution (narrow ethoxylation degree).

However, one may also start from commercially available ethoxylated alcohols.

In the second step, the ethoxylated alcohols are reacted with a strong base, like sodium or potassium hydroxide, in presence of a reducing agent, i.e. sodium borohydride, to obtain the corresponding alkoxylate, which is carboxymethylated with sodium monochloroacetate (SMCA).

The conversion of the ethoxylated alcohols to the ether carboxylates depends, among other things, on the temperature, the reaction time, the molar excess of sodium monochloroacetate/NaOH to the ethoxylated alcohols, the water content during the reaction and the ethoxylation degree of the alcohols (normally the higher the ethoxylation degree the higher the conversion to the ether carboxylate)

According to the invention, it is preferred that the average degree of carboxymethylation of the alkyl ether carboxylates is in the range from 0.3 to 0.75, preferably from 0.4 to 0.6, so that the composition contains, besides the components (a) and (b), component (c), ethoxylated fatty alcohols of formula II

R-O-(CH₂CH₂O)ₙ-H Formula II

wherein
R is an alkyl residue having 6 to 22 carbon atoms;
n has an average value in the range of 2.0 to 4.5.

Fatty alcohols of the Formula II are thus preferably present in a mole ratio of alkyl ether carboxylates (b) / ethoxylated fatty alcohols (c) of 3:7 to 7.5:2.5, more preferably 4:6 to 6:4, most preferably 1:1. The weight ratio of ethoxylated fatty alcohol (c) / glycerol (b) is preferably in the same range as the weight ratio of alkyl ether carboxylate (a) to glycerol (b).

According to the invention, to the crude alkyl ether carboxylate obtained as described above, glycerol is added and finally the pH is adjusted to 5.5-6.5, e.g. with water and hydrochloric acid or sulphuric acid.

Optionally, the crude alkyl ether carboxylate mixture can be purified in order to remove sodium chloride and other by-products to the extent that is possible. This can be carried out by stirring the reaction mixture with water and hydrochloric acid or sulphuric acid and by heating to 80-100°C. After stopping the stirrer, an oil layer (alkyl ether carboxylic acid) and a high salt-water layer are formed. These two layers are separated and after that glycerol is added to the alkyl ether carboxylic acid and finally the pH is adjusted to 5.5-6.5.

It is preferred that the weight ratio of ethoxylated fatty alcohol (prior to carboxymethylation) to glycerol (component b) is in the range of 5:1 to 15:1, preferably in the range from 8:1 to 13:1, more preferably in the range from 9:1 to 12:1.

The total amount of glycerol (component b) is in the range of 0.5 to 15 wt.%, preferably 3 to 9 wt.% based on the total weight of the composition.

Glycerol (CH₂OHCHOHCH₂OH) is a colorless, odorless, sweet-tasting, syrupy liquid. Glycerol is a trihydric alcohol which melts around 17-18°C, boils with decomposition at 290°C, and is miscible with water and ethanol. It is hygroscopic; i.e., it absorbs water from the air; this property makes it valuable as a moisturizer in cosmetics. Glycerol is present in the form of its esters (glycerides) in all animal and vegetable fats and oils. It is obtained commercially as a by-product when fats and oils are hydrolyzed to yield fatty acids or their metal salts (soaps). Glycerol is also synthesized on a commercial scale from propylene (obtained by cracking petroleum). Glycerol can also be obtained during the fermentation of sugars if sodium bisulfite is added with the yeast.

Preferably, the aqueous composition according to the invention comprises, based on the total weight of the composition,
(i) from 20 to 45 wt.% of alkyl ether carboxylate (component a)
(ii) from 3 to 9 wt.% of glycerol (component b)
(iii) from 20 to 45 wt.% of ethoxylated fatty alcohol (component c)

The amount of water to be added to the mixture of compounds (a) and (b) and/or to the mixture of compounds (a), (b) and (c) in order to obtain a homogeneous liquid aqueous solution, pumpable at room temperature, is, as a rule, at most 55% (weight relative to the weight of the solution), and preferably at most 45 wt.%, even more preferred at most 40 wt.%. A larger amount of water may also be added, but then the advantages of the solution being concentrated will be lost. Also more diluted solutions require the use of preservatives against microbiological contamination.

The aqueous composition of the present invention may be used to improve the foam properties anionic, cationic, non-ionic and/or amphoteric surfactants or mixtures thereof in the form of aqueous solution. Those aqueous compositions which are used to enhance the foaming of other surfactant compositions (in the following referred to as "foam booster") preferably contain at least 45 wt.%, more preferably at least 50 wt.%, of components (a), i.e. the ethercarboxylate, (b), i.e. the glycerol, and (c), i.e. the ethoxylated fatty alcohol, as defined above. The amount of components (a) and (c) is preferably in the range of 20 to 45 wt.%, more preferably 14 to 30 wt.%. The amount of component (b) is preferably in the range of 0.5 to 15 wt.%, more preferably 3 to 9 wt.%. The active matter content of the foam booster is also preferably at least 45 wt.%, more preferably at least 50 wt.%, the active matter of the composition is substantially provided by components (a), (b) and (c). The foam booster of the present invention is flowable at room temperature.

While the foam booster thus does not include significant amounts of other surfactants, it may include sodium chloride and impurities, as a result of the preparation process of the alkyl ether carboxylates. The foam booster of the present invention generally do not contain a peaked (narrow range) short chain alcohols since this would unnecessarily increase the costs.

The foam booster according to the invention is used for the production of personal hygiene and/or personal care cosmetic compositions. The present invention also provides a personal hygiene and/or personal care cosmetic composition, comprising the foam booster of the present invention (i.e. the aqueous composition as defined above) in which the components (a), (b) and optionally (c) are present in quantities of 0.5 to 25, preferably 1 to 20, even more preferably 2 to 10, more particularly 2 to 7% by weight, based on the cosmetic composition. These cosmetic compositions generally contain, in addition to components (a) and (c), anionic, cationic, non-ionic or amphoteric surfactants, or mixtures thereof. These additional surfactants are generally present so that the total amount of surfactants does not exceed 40 wt.%. The preferred total amount is in the range of 10 to 35 wt.%.

The cosmetic compositions are, for example, liquid soaps, hair shampoos, body shampoos, hair lotions, foam baths, shower baths, creams, gels, lotions, shaving preparations, after-shaving preparations, alcoholic and aqueous/alcoholic solutions or emulsions.

Examples of these additional surfactants include the following.

### Anionic surfactants

Typical examples of anionic surfactants are soaps, preferably C₁₂-C₁₈ fatty acid soaps, like soaps derived from lauric acid, myristic acid, palmitic acid, stearic acid, isostearic acid, oleic acid or linoleic acid. Said soaps contain an appropriate cation, selected from the group consisting of an alkali metal, an alkaline earth metal, ammonium, an alkylammonium, an alkanolammonium or a glucammonium. Preferably the cation group is selected from the group consisting of sodium, potassium and triethanolamine. Preferred C₁₂-C₁₈ fatty acid soaps include triethanolamine stearate, triethanolamine palmitate, triethanolamine myristate, triethanolamine laurate, sodium stearate, sodium palmitate, sodium myristate, sodium laurate, potassium stearate, potassium palmitate, potassium myristate, and potassium laurate.

Other examples of anionic surfactants are alkylbenzenesulfonates, alkanesulfonates, olefin sulfonates, alkylether sulfonates, glycerol ether sulfonates, α-methyl ester sulfonates, sulfofatty acids, alkylsulfates, fatty alcohol ether sulfates, glycerol ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, amide ether carboxylic acids and salts thereof, fatty acid isethionates, fatty acid sarcosinates, fatty acid taurides, N-acylamino acids such as, for example, acyl lactylates, acyl tartrates, acyl glutamates, acyl aspartates, alkyl oligoglucoside sulfates, protein fatty acid condensates (particularly wheat-based vegetable products) and alkyl (ether) phosphates.

One of the preferred anionic surfactant is sodium lauryl ether sulfate, particularly preferred is sodium lauryl ether sulfate having an average ethoxylation degree of 1 to 3, preferably 1 to 2.5, most preferably 2 to 2.5.

### Cationic surfactants

Typical examples of cationic surfactants are amine salts, quaternary ammonium salts (quats) like monoalkyl dimethyl amine derivatives, dialkyl monomethyl amines and imidazoline derivatives, and the quaternized derivatives of polyalkanolamine esters (esterquats). Examples of commercially available quats are: Quartamin® AB (Behentrimonium Chloride), Quartamin® 60W25 (Cetrimonium Chloride) and Quartamin® ABK (Behentrimonium Chloride and Cetearyl Alcohol), all marketed by KAO Corporation S.A.

Examples of commercially available esterquats are Quartamin® BTC-131 (Behenoyl PG-Trimonium Chloride), marketed by KAO Chemicals GmbH, and Tetranyl ® CO-40 (Dioleoylethyl Hydroxyethylmonium Methosulfate and Dipropylene Glycol) marketed by KAO Corporation S.A.

### Non-ionic surfactants

Specific examples of non-ionic surfactants are alkoxylated trimethyolol propane, alkoxylated 1,2,3-trihydroxy hexane, alkoxylated pentaetrythritol, alkoxylated sorbitol, alkoxylated glycerol fatty acid ester, alkoxylated trimethyolol propane fatty acid ester, alkoxylated 1,2,3-trihydroxy hexane fatty acid ester, alkoxylated pentaetrythritol fatty acid ester, alkoxylated sorbitol fatty acid ester, fatty alcohol polyglycol ethers, alkylphenol polyglycol ethers, fatty acid polyglycol esters, fatty acid amide polyglycol ethers, fatty amine polyglycol ethers, mixed ethers and mixed formals, optionally partly oxidized alk(en)yl oligoglycosides or glucuronic acid derivatives, fatty acid-N-alkylglucamides, protein hydrolyzates (particularly wheat-based vegetable products), polyol fatty acid esters, sugar esters, alkyl polyglucosides, sorbitan esters, polysorbates. and alkanolamides, including alkoxylated alkanolamides, preferably ethoxylated alkanolamides derived from rapeseed oil, and alkyl ether carboxylic acid alkanolamides, preferably ethoxylated alkyl C11-C13 carboxylic acid alkanolamides.

### Amphoteric surfactants

Specific examples of amphoteric surfactant are alkyl amine oxides, alkyl betaines, alkyl sulphobetaines (sultaines), amidoalkyl betaines, alkyl glycinates, alkyl carboxyglycinates, alkyl amphoacetates, alkyl amphopropionates, alkylamphoglycinates, alkyl amidopropyl betaines, alkyl amidopropyl- and hydroxysultaines. Particularly preferred amphoteric surfactants are alkyl amine oxides, alkyl sulphobetaines (sultaines), alkylamphoglycinates alkyl amphoacetates such as sodium coco monoamphoacetate or sodium coco diamphoacetate, and alkyl amidopropyl betaines such as cocoamido propyl betaine.

The weight ratio of said anionic, non-ionic, amphoteric or cationic surfactant, preferably anionic surfactant, to the total weight of components (a), (b) and, if present, (c) is preferably in the range of 2:1 to 6:1, more preferably 3:1 to 5:1.

### Other components

These cosmetic compositions may also contain mild surfactants, oil components, superfatting agents, pearlizing waxes, consistency factors, thickeners, polymers, silicone compounds, fats, waxes, stabilizers, biogenic agents, deodorizers, anti-dandruff agents, film formers, swelling agents, UV protection factors, antioxidants, hydrotropes, preservatives, insect repellents, self-tanning agents, solubilizers, perfume oils, dyes, germ inhibitors and the like as further auxiliaries and additives.

### Co-emulsifiers

In one preferred embodiment of the invention, the preparations may contain other anionic, nonionic, cationic, amphoteric and/or zwitterionic co-emulsifiers as an optional component (d), their percentage content-based on the preparation--being from 0.5 to 10, preferably from 1 to 8 and more particularly from 2 to 5% by weight. Suitable co-emulsifiers are, for example, nonionic surfactants from at least one of the following groups:
(1) products of the addition of 2 to 30 moles of ethylene oxide and/or 0 to 5 moles of propylene oxide onto linear fatty alcohols containing 8 to 22 carbon atoms, onto fatty acids containing 12 to 22 carbon atoms and onto alkylphenols containing 8 to 15 carbon atoms in the alkyl group;
(2) ethoxylated glycerides as described in the European patent applications EP-A-0586323 and EP-A-1045021, preferably obtained by reaction of triglycerides, glycerine and ethylene oxide;
(3) glycerol monoesters and diesters and sorbitan monoesters and diesters of saturated and unsaturated fatty acids containing 6 to 22 carbon atoms and ethylene oxide adducts thereof;
(4) alkyl mono- and oligoglycosides containing 8 to 22 carbon atoms in the alkyl group and ethoxylated analogs thereof;
(5) products of the addition of 15 to 60 moles of ethylene oxide onto castor oil and/or hydrogenated castor oil;
(6) polyol esters and, in particular, polyglycerol esters such as, for example, polyglycerol polyricinoleate, polyglycerol poly-12-hydroxy-stearate or polyglycerol dimerate isostearate. Mixtures of compounds from several of these classes are also suitable;
(7) products of the addition of 2 to 15 moles of ethylene oxide onto castor oil and/or hydrogenated castor oil;
(8) partial esters based on linear, branched, unsaturated or saturated C6-22 fatty acids, ricinoleic acid and 12-hydroxystearic acid and glycerol, polyglycerol, pentaerythritol, dipentaerythritol, sugar alcohols (for example sorbitol), alkyl glucosides (for example methyl glucoside, butyl glucoside, lauryl glucoside) and polyglucosides (for example cellulose);
(9) mono-, di- and trialkyl phosphates and mono-, di-and/or tri-PEG-alkyl phosphates and salts thereof;
(10) wool wax alcohols;
(11) polysiloxane/polyalkyl polyether copolymers and corresponding derivatives;
(12) mixed esters of pentaerythritol, fatty acids, citric acid and fatty alcohol and/or mixed esters of fatty acids containing 6 to 22 carbon atoms, methyl glucose and polyols, preferably glycerol or polyglycerol,
(13) polyalkylene glycols and
(14) glycerol carbonate.

The addition products of ethylene oxide and/or propylene oxide with fatty alcohols, fatty acids, alkylphenols, glycerol mono- and diesters and sorbitan mono- and diesters of fatty acids or with castor oil are known commercially available products. They are homolog mixtures of which the average degree of alkoxylation corresponds to the ratio between the quantities of ethylene oxide and/or propylene oxide and substrate with which the addition reaction is carried out.

Ethoxylated glycerides according to the invention are preferably a mixture of compounds of the following formula 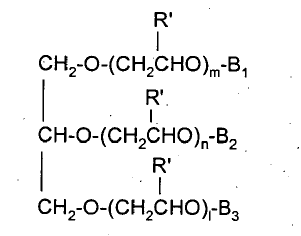 wherein
R' represents H or CH₃;
each m, n, and l independently represent a number from 0 to 40, the sum of m, n and 1 being in the range of 1 to 100, preferably 1 to 20, and said mixtures comprising
(i) compounds represented by formula (I), where one of B1, B2 and B3 represents an acyl group having 6 to 22 carbon atoms, the remainder representing H;
(ii) compounds represented by formula (I), where two of B1, B2 and B3, independently, represent an acyl group having 6 to 22 carbon atoms, the remainder representing H;
(iii) compounds represented by formula (I), where each B1, B2 and B3, independently, represent an acyl group having 6 to 22 carbon atoms;
(iv) compounds represented by formula (I), where each of B1, B2 and B3 represent H;
the weight ratio of the compounds (i) / (ii) /(iii) being 46-90/9-35/1-15. Particularly preferred are compounds of the formula III wherein the weight ratio (i)+(ii)+(iii)/(iv) is in the range of 85/15 to 40/60, more preferably in the range 80/20 to 45/55.

C₈-₁₈ alkyl mono- and oligoglycosides, their production and their use as surfactants are known from the art. They are produced in particular by reaction of glucose or oligosaccharides with primary alcohols containing 8 to 18 C atoms. So far as the glycoside component is concerned, both monoglycosides, in which a cyclic sugar unit is attached to the fatty alcohol by a glycoside linkage, and oligomeric glycosides with a degree of oligomerization of preferably up to about 8 are suitable. The degree of oligomerization is a statistical mean value on which a homolog distribution typical of such technical products is based.

Zwitterionic surfactants may also be used as emulsifiers. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known by the CTFA name of Cocamidopropyl Betaine is particularly preferred. Other suitable emulsifiers are ampholytic surfactants. Ampholytic surfactants are surface-active compounds which, in addition to a C8-18 alkyl or acyl group, contain at least one free amino group and at least one -COOH or ―SO₃H group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and C₁₂₋₁₈ acyl sarcosine. Suitable anionic emulsifiers are, in particular, alkyl (ether) sulfates, acyl glutamates, protein fatty acid condensates and monoglyceride sulfates. Besides ampholytic emulsifiers, quaternary emulsifiers may also be used, those of the esterquat type, preferably methyl-quaternized difatty acid triethanolamine ester salts, being particularly preferred.

Suitable oil components are, for example, Guerbet alcohols based on fatty alcohols containing 6 to 18 and preferably 8 to 10 carbon atoms, esters of linear C₆-C₂₂ fatty acids with linear C₆-C₂₂ fatty alcohols, esters of branched C6-13 carboxylic acids with linear C₆-C₂₂ fatty alcohols such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂ fatty acids with branched alcohols, more particularly 2-ethyl hexanol, esters of hydroxycarboxylic acids with linear or branched C₆-C₂₂ fatty alcohols, more especially Dioctyl Malate, esters of linear and/or branched fatty acids with polyhydric alcohols (for example propylene glycol, dimer diol or trimer triol) and/or Guerbet alcohols, triglycerides based on C₆-C₁₀ fatty acids, liquid mono-/di-/triglyceride mixtures based on C₆-C₁₈ fatty acids, esters of C₆-C₂₂ fatty alcohols and/or Guerbet alcohols with aromatic carboxylic acids, more particularly benzoic acid, esters of C₆-C₁₂ dicarboxylic acids with linear or branched alcohols containing 1 to 22 carbon atoms or polyols containing 2 to 10 carbon atoms and 2 to 6 hydroxyl groups, vegetable oils, branched primary alcohols, substituted cyclohexanes, linear and branched C₆-C₂₂ fatty alcohol carbonates, Guerbet carbonates, esters of benzoic acid with linear and/or branched C₆-C₂₂ alcohols, linear or branched, symmetrical or nonsymmetrical dialkyl ethers containing 6 to 22 carbon atoms per alkyl group, ring opening products of epoxidized fatty acid esters with polyols, silicone oils and/or aliphatic or naphthenic hydrocarbons, for example squalane, squalene or dialkyl cyclohexanes.

Suitable secondary consistency factors are hydroxyfatty alcohols, partial glycerides, fatty acids or hydroxyfatty acids. Suitable thickeners are, for example, Aerosil types (hydrophilic silicas), polysaccharides, more particularly xanthan gum, guar guar, agar agar, alginates and tyloses, carboxymethyl cellulose and hydroxyethyl cellulose, relatively high molecular weight polyethylene glycol monoesters and diesters of fatty acids, polyacrylates (for example Carbopols [Goodrich] or Synthalens [Sigma]), polyacrylamides, polyvinyl alcohol and polyvinyl pyrrolidone, surfactants such as, for example, ethoxylated fatty acid glycerides, esters of fatty acids with polyols such as, for example, pentaerythritol or trimethylol propane, narrow-range fatty alcohol ethoxylates or alkyl oligoglucosides and electrolytes, such as sodium chloride and ammonium chloride.

Suitable cationic polymers are, for example, cationic cellulose derivatives such as, for example, the quaternized hydroxyethyl cellulose obtainable from Amerchol under the name of Polymer JR 400, cationic starch, copolymers of diallyl ammonium salts and acrylamides, quaternized vinyl pyrrolidone/vinyl imidazole polymers such as, for example, Luviquat (BASF), condensation products of polyglycols and amines, quaternized collagen polypeptides such as, for example, Lauryidimonium Hydroxypropyl Hydrolyzed Collagen (Lamequat L, Grunau), quaternized wheat polypeptides, polyethyleneimine, cationic silicone polymers such as, for example, Amodimethicone, copolymers of adipic acid and dimethylamino-hydroxypropyl diethylenetriamine (Cartaretine , Sandoz), copolymers of acrylic acid with dimethyl diallyl ammonium chloride (Merquat 550, Chemviron), polyaminopolyamides and crosslinked water-soluble polymers thereof, cationic chitin derivatives such as, for example, quaternized chitosan, optionally in micro-crystalline distribution, condensation products of dihaloalkyls, for example dibromobutane, with bis-dialkylamines, for example bis-dimethylamino-1,3-propane, cationic guar gum.such as, for example, Jaguar CBS, Jaguar C-17, Jaguar C-16 of Celanese, quaternized ammonium salt polymers such as, for example, Mirapol A-15, Mirapol AD-1, Mirapol AZ-1 of Miranol.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones.

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Preferred silicone compounds are hydrophobic silicone oils, which are silicone oils which are soluble in paraffinic oil at 25°C. Hydrophobic silicone oils to be used according to the present invention include both volatile and non-volatile silicone oils.

Specific examples include a cyclic methyl siloxane having the formula {(CH₃)₂SiO}ₓ in which x is 3-6, or short chain linear methyl siloxanes having the formula ((CH₃)₂SiO{(CH₃)₂SiO}_{y}Si(CH₃)₃ in which y is 0-5.

Some suitable cyclic methyl siloxanes are hexamethylcyclotrisiloxanes (D₃), a solid with a boiling point of 134°C and the formula {(Me₂)SiO}₃; octamethylcyclotetrasiloxane (D₄) with a boiling point of 176°C, a viscosity of 2.3 mm²/s, and the formula {(Me₂)SiO}₄; decamethylcyclopentasiloxane (D₅) (cyclomethicone) with a boiling point of 210°C, a viscosity of 3.87 mm²/s, and the formula {(Me₂)SiO}₅; and dodecamethylcyclohexasiloxane (D₆) with a boiling point of 245°C, a viscosity,of 6.62 mm²/s and the formula {(Me₂)SiO}₆.

Some suitable short linear methyl siloxane are hexamethyldisiloxane (MM) with a boiling point of 100°C, viscosity of 0-65 mm²/s, and formula Me₃SiOMe₃; octamethyltrisiloxane (MDM) with a boiling point of 152°C, viscosity of 1.04 mm²/s, and formula Me₃SiOMe₂SiOSiMe₃; decamethyltetrasiloxane (MD2M) with a boiling point of 194°C, viscosity of 1.53 mm²/s, and formula Me₃SiO(MeSiO)₂SiMe₃; dodecamethylpentasiloxane (MD3M) with a boiling point of 229°C, viscosity of 2.06 mm²/s, and formula Me₃SiO(Me₂SiO)₃SiMe₃; tetradecamethylhexasiloxane (MD4M) with a boiling point of 245°C, viscosity of 2.63 mm²/s, and formula Me₃SiO(Me₂SiO)₄SiMe₃; and hexadecamethylheptasiloxane (MD5M) with a boiling point of 270°C, viscosity of 3.24 mm²/s, and formula Me₃SiO(Me₂SiO)₅SiMe₃.

Furthermore, long chain linear siloxanes such as phenyltrimethicone, bis(phenylpropyl)dimethicone, dimethicone, and dimethiconol are also included.

Typical examples of fats are glycerides while suitable waxes are inter alia natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes, microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes.

Metal salts of fatty acids such as, for example, magnesium, aluminium and/or zinc stearate or ricinoleate may be used as stabilizers.

In the context of the invention, biogenic agents are, for example, tocopherol, tocopherol acetate, tocopherol palmitate, ascorbic acid, deoxyribonucleic acid, retinol, bisabolol, allantoin, phytantriol, panthenol, AHA acids, amino acids, ceramides, pseudoceramides, essential oils, plant extracts and vitamin complexes.

Suitable deodorizers are, for example, antiperspirants, such as aluminium chlorhydrates. These antiperspirants are colorless hygroscopic crystals which readily deliquesce in air and which accumulate when aqueous aluminium chloride solutions are concentrated by evaporation. Besides the chlorhydrates, aluminium hydroxylactates and acidic aluminium/zirconium salts may also be used. Other suitable deodorizers are esterase inhibitors, preferably trialkyl citrates, such as trimethyl citrate, tripropyl citrate, triisopropyl citrate, tributyl citrate and, in particular, triethyl citrate. Esterase inhibitors inhibit enzyme activity and thus reduce odor formation. The free acid is probably released through the cleavage of the citric acid ester, reducing the pH value of the skin to such an extent that the enzymes are inhibited. Other esterase inhibitors are sterol sulfates or phosphates, for example lanosterol, cholesterol, campesterol, stigmasterol and sitosterol sulfate or phosphate, dicarboxylic acids and esters thereof, for example glutaric acid, glutaric acid monoethyl ester, glutaric acid diethyl ester, adipic acid, adipic acid monoethyl ester, adipic acid diethyl ester, malonic acid and malonic acid diethyl ester, hydroxycarboxylic acids and esters thereof, for example citric acid, malic acid, tartaric acid or tartaric acid diethyl ester. Antibacterial agents which influence the germ flora and destroy or inhibit the growth of perspiration-decomposing bacteria, may also be present in stick products. Examples of such antibacterial agents are chitosan, phenoxyethanol and chlorhexidine gluconate. 5-Chloro-2-(2,4-dichlorophenoxy)-phenol.

Suitable antidandruff agents are climbazol, octopirox and zinc pyrithione. Standard film formers are, for example, chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds. Suitable swelling agents for aqueous phases are montmorillonites, clay minerals, Pemulen and alkyl-modified Carbopol types (Goodrich).

Examples of UV protection factors include organic substances (light filters) which are liquid or crystalline at room temperature and which are capable of absorbing ultraviolet radiation and of releasing the energy absorbed in the form of longer-wave radiation, for example heat. UV-B filters can be oil-soluble or water-soluble. The following are examples of oil-soluble substances:
- 3-benzylidene camphor or 3-benzylidene norcamphor and derivatives thereof, for example 3-(4-methylbenzylidene)-camphor;
- 4-aminobenzoic acid derivatives, preferably 4-(dimethylamino)-benzoic acid-2-ethylhexyl ester, 4-(dimethylamino)-benzoic acid-2-octyl ester and 4-(dimethylamirio)-benzbic acid amyl ester; esters of cinnamic acid, preferably 4-methoxycinnamic acid-2-ethylhexyl ester, 4-methoxycinnamic acid propyl ester, 4-methoxycinnamic acid isoamyl ester, 2-cyano-3,3-phenylcinnamic acid-2-ethylhexyl ester (Octocrylene);
- esters of salicylic acid, preferably salicylic acid-2-ethylhexyl ester, salicylic acid-4-isopropylbenzyl ester, salicylic acid homomenthyl ester;
   derivatives of benzophenone, preferably 2-hydroxy-4-methoxybenzo-phenone, 2-hydroxy-4-methoxy-4'-methylbenzophenone, 2,2'-dihydroxy-4-methoxybenzophenone;
- esters of benzalmalonic acid, preferably 4-methoxybenzalmalonic acid di-2-ethylhexyl ester;
- triazine derivatives such as, for example, 2,4,6-trianilino-(p-carbo-2'-ethyl-1'-hexyloxy)-1, 3,5-triazine and Octyl Triazone;
- propane-1,3-diones such as, for example, 1-(4-tert.butylphenyl)-3-(4'-methoxyphenyl)-propane-1 , 3-dione;
- 2-phenylbenzimidazole-5-sulfonic acid and alkali metal, alkaline earth metal, ammonium, alkylammonium, alkanolammonium and glucam-monium salts thereof;
- sulfonic acid derivatives of benzophenones, preferably 2-hydroxy-4-methoxybenzophenone-5-sulfonic acid and salts thereof;
- sulfonic acid derivatives of 3-benzylidene camphor such as, for example, 4-(2-oxo-3-bornylidenemethyl)-benzene sulfonic acid and 2-methyl-5-(2-oxo-3-bornylidene)-sulfonic acid and salts thereof.

Typical UV-A filters are, in particular, derivatives of benzoyl methane such as, for example 1-(4'-tert.butylphenyl)-3-(4'-methoxyphenyl)-propane-1,3-dione, 4-tert-butyl-4'-methoxydibenzoylmethane (Parsol 1789) or 1-phenyl-3-(4'-isopropylphenyl)-propane-1,3-dione.

The UV-A and UV-B filters may of course also be used in the form of mixtures. Besides the soluble substances mentioned, insoluble pigments, i.e. finely dispersed metal oxides or salts, may also be used for this purpose. Examples of suitable metal oxides are, in particular, zinc oxide and titanium dioxide and also oxides of iron, zirconium, silicon, manganese, aluminium and cerium and mixtures thereof. Silicates (talcum), barium sulfate and zinc stearate may be used as salts. The oxides and salts are used in the form of the pigments for skin-care and skin-protecting emulsions and decorative cosmetics. The particles should have an average diameter of less than 100 nm, preferably from 5 to 50 nm and more preferably from 15 to 30 nm. They may be spherical in shape although ellipsoidal particles or other non-spherical particles may also be used. The pigments may also be surface-treated, i.e. hydrophilicized or hydrophobicized. Typical examples are coated titanium dioxides such as, for example, Titandioxid T 805 (Degussa) or Eusolex T2000 (Merck). Suitable hydrophobic coating materials are, above all, silicones and especially trialkoxyoctyl silanes or simethicones. So-called micro- or nanopigments are preferably used in sun protection products. Micronized zinc oxide is preferably used.

Besides the two above-mentioned groups of primary protection factors, secondary protection factors of the antioxidant type may also be used. Secondary sun protection factors of the antioxidant type interrupt the photochemical reaction chain which is initiated when UV rays penetrate into the skin. Typical examples of suitable antioxidants are amino acids (for example glycine, histidine, tyrosine, tryptophane) and derivatives thereof, imidazoles (for example.urocanic acid) and derivatives thereof, peptides, such as D,L-carnosine, D-carnosine, L-carnosine and derivatives thereof (for example anserine), carotinoids, carotenes (for example α-carotene, β-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, γ-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages (also (metal) chelators (for example (α-hydroxyfatty acids, palmitic acid, phytic acid, lactoferrine), α-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example γ-linolenic acid, linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, (α-glycosyl rutin, ferulic acid, furfurylidene glucitol, carnosine, butyl hydroxytoluene, butyl hydroxyanisole, nordihydroguaiac resin acid, nordihydroguaiaretic acid, trihydroxy-butyrophenone, uric acid and derivatives thereof, mannose and derivatives thereof, Superoxid-Dismutase, zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

In addition, hydrotropes such as, for example, ethanol, isopropyl alcohol or polyols may be used to improve flow behavior. Suitable polyols preferably contain 2 to 15 carbon atoms and at least two hydroxyl groups. The polyols may contain other functional groups, especially amino groups, or may be modified with nitrogen. Typical examples are
- alkylene glycols such as, for example, ethylene glycol, diethylene glycol, propylene glycol, butylene glycol, hexylene glycol and polyethylene glycols having an average molecular weight of 100 to 1,000 dalton;
- technical oligoglycerol mixtures with a degree of self-condensation of 1.5 to 10 such as, for example, technical diglycerol mixtures with a diglycerol content of 40 to 50% by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms such as, for example, sorbitol or mannitol;
- sugars containing 5 to 12 carbon atoms such as, for example, glucose or sucrose;
- aminosugars such as, for example, glucamine;
- dialcoholamines, such as diethanolamine or 2-aminopropane-1,3-diol.

Suitable preservatives are, for example, phenoxyethanol, formaldehyde solution, parabens, pentanediol or sorbic acid. Suitable insect repellents are N,N-diethyl-m-toluamide, pentane-1,2-diol or Insect Repellent 3535. A suitable self-tanning agent is dihydroxyacetone.

Suitable perfume oils are mixtures of natural and synthetic fragrances. Natural fragrances include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamon, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert-butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, α-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable fragrance. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, labolanum oil and lavendin oil. The following are preferably used either individually or in the form of mixtures: bergamot oil, dihydromyrcenol, lilial, lyral, citronellol, phenylethyl alcohol, α-hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, β-damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

Suitable dyes are any of the substances suitable and approved for cosmetic purposes. These dyes are normally used in concentrations of 0.001 to 0.1% by weight, based on the mixture as a whole.

Typical examples of germ inhibitors are preservatives which act specifically against gram-positive bacteria such as, for example, 2,4,4'-trichloro-2'-hydroxydiphenyl ether, chlorhexidine (1,6-di-(4-chlorophenyl-biguanido)-hexane) or TCC (3,4,4'-trichlorocarbanilide). Numerous perfumes and essential oils also have antimicrobial properties. Typical examples are the active substances eugenol, menthol and thymol in clove, mint and thyme oil. The percentage content of the additional germ-inhibiting agents is normally about 0.1 to 2% by weight, based on the solids component of the preparations.

The total percentage content of auxiliaries and additives may be from 1 to 50% by weight and is preferably from 5 to 40% by weight, based on the particular composition. The preparations may be produced by standard hot or cold processes.

If the cosmetic compositions are hair shampoos or body shampoos the total amount of wash active matter, that is, the total amounts of surfactants contained in the composition of the present invention is preferably less than 30 wt.-%. That is, if the composition contains surfactants other than components (a), (b) and (c), and the total amount of these surfactants and components (a), (b) and (c) and does desirably not exceed 30 wt.-%.

It is particularly preferred to employ the foam booster of the present invention in cosmetic or cleaning preparations for baby skin, including baby shampoo; dry skin; facial skin; in anti-aging compositions, compositions for the treatment of psoriasis or seborrhoea; or in shower baths. These compositions oftentimes have a comparably low amount of ether sulfate (less than 5 wt.%) and further comprise protein derivatives, acyl glutamates, amphoacetates, moisturizers such as Glycereth-2 cocoate, Glycereth-7 cocoate, or vegetable oil.

The following examples are given in order to provide a person skilled in the art with a sufficiently clear and complete explanation of the present invention, but should not be considered as limiting of the essential aspects of its subject, as set out in the preceding portions of this description.

### Examples

### 1. Preparation of alkyl ether carboxylates

### 1.a) Composition 1

A composition according to the invention is prepared following the process described here below:
1. 2809.5 g of lauryl alcohol (15.105 mole) were condensated with 1994.0 g of ethylene oxide (45.318 mole) in a stainless steel autoclave at 130°C in the presence of potassium hydroxide 91% (KOH) flakes as catalyst obtaining an ethoxylated lauryl alcohol (3 EO).
2. 729.44 g (2.294 mole) of said ethoxylated lauryl alcohol (3 EO) are carboxymethylated with 133.62 g (1.147 mole) of sodium monochloroacetate (SMCA) in the presence of 48.63 g (1.216 mole) of sodium hydroxide prills. The product (approximately 50% of carboxymethylation) was a white liquid product at room temperature.
3. The chloride content is measured (potentiometric) to be sure that the reaction with the SMCA is completed.
4. To 924.9 g of this product, 72.9 g of glycerol is added. Then the alkaline value is measured with 0.1 M HCl solution. The pH is adjusted with 32 wt.% HCl solution.
5. The product is diluted by water (455.7 g) to a NaCl content of about 5 wt.% (based on the total amount of the composition)
This leads to a composition having an active matter of 60 wt.% and a water content of 35 wt.%, having a viscosity of 28,500 mPa.s at 20°C and a pH of 6.0.

### 1.b) Composition C1 (comparative experiment)

The same process was repeated but without the addition of glycerol. The water was adjusted in order to have a water content of 35 wt.%. Said product was a paste.

### 1.c) Composition C2 (comparative experiment)

The same process was repeated for obtaining an alkyl ether carboxylate having an average ethoxylation degree of 4.5, approximately 80% of carboxymethylation and without the addition of glycerol. It was diluted with water to a water content of 78 wt.%. Said product was liquid.

### 1.d) Composition C3 (comparative experiment)

The same process was repeated but replacing glycerol by ethanol. Said product was a liquid under agitation, but at rest it turned into a thick gel.

### 2. Viscosity values

The viscosity of the compounds/compositions indicated in Table 1 was measured at 20°C with a Brookfield viscometer LVT (supplied by Brookfield Engineering Laboratories Inc. Stoughton, MA, USA) in accordance with DIN 1341 (spindle 2 at 30 rpm for viscosities in the range of up to 1,000 mPa.s; spindle 3 at 12 rpm for viscosities in the range of 1,000 to 7,000 mPa.s; spindle 4 at 12 rpm for viscosities in the range of more than 7,000 mPa.s)

**Table 1 - Viscosity Values**

| Aqueous composition | | (a) | | (b) | Water (wt.%) | Viscosity (mPa.s) |
|---|---|---|---|---|---|---|
| | | Sodium lauryl ether carboxylate | | | | |
| | | EO degree | Carboxymethylation degree | | | |
| Invention | 1 | 3.0 | 50 % | Glycerol | 35 % | 28,500 |
| Comparative Experiments | C1 | 3.0 | 50 % | | 35 % | paste |
| | C2 | 4.5 | 80 % | | 78 % | 10-20 |
| | C3 | 3.0 | 50 % | Ethanol | 35 % | 60,000 |
| | C4 | Akypo Soft 100 BVC¹ | | | 30 % | 1,000 |
| | C5 | Akypo Soft 70 BVC² | | | 30 % | 52,000 |

| | | | | | | |
|---|---|---|---|---|---|---|
| ¹Mixture comprising 30 to 40 wt.% Sodium Laureth-11 Carboxylate, 20 to 30 wt.% Laureth-10 and 5 to 10 wt.% ethoxylated glycerine and carboxymethylated products thereof, the balance being water and sodium chloride, marketed by Kao Chemicals GmbH ²Mixture comprising 30 to 40 wt.% Sodium Laureth-8 Carboxylate, 20 to 30 wt.% Laureth-7 and 5 to 10 wt.% ethoxylated glycerine and carboxymethylated products thereof, the balance being water and sodium chloride, marketed by Kao Chemicals GmbH | | | | | | |

### 3. Foaming power

Aqueous surfactant solutions (15 wt.% a.m.) containing sodium laureth-2 sulfate and different compounds in a weight ratio 3:1 (based on active matter) were prepared. The foaming power of 0.1 wt.-% active matter of said surfactant solutions shown in Table 2 below (pH 6-7; 40°C; 15°dH) was determined according to the Reverse Stirring Agitation" method^{a} in the presence of 0.5 wt.-% "Spangler sebum"^{b}.

This method consists in measuring the foam volume after agitation of 100 g of a diluted solution of the test product, placed into a 400 mL'graduated cylinder, the cylinder being attached at its middle section to the axle of a motor and is rotated at 1100 rpm. with a metallic stirrer on a vertical plane perpendicular to this axle, changing the direction every 6 seconds during 5 minutes.

The measures were taken after 30 seconds in duplicate. The values shown in Table 2 are the average of the obtained results.

**Table 2 - Foam Values**

| | Surfactant compositions | Mixture SLES/Foam booster 3:1 (based on active matter) | | Foaming Power (mL) after 30 s |
|---|---|---|---|---|
| Invention | S1 | SLES | Aqueous compos. 1 | 115 |
| Comparative Experiments | SC1 | SLES | C1 | 105 |
| | SC2 | SLES | C2 | 71 |
| | SC3 | SLES | C3 | 114 |
| | SC4 | SLES | C4 | 27 |
| | SC5 | SLES | C5 | 32 |
| | SC6 | SLES | | 91 |
| | SC7 | SLES | Glycerol | 82 |
| | SC8 | SLES | Cocoamidopropyl betaine | 105 |

| | | | | |
|---|---|---|---|---|
| ^{a} (Castán, P.; Winkel, S.; Amela, C.; Garcia, A.; Siscart, N.; Kao Corporation, S.A., Spain.; "A brief study about foams. Foaming properties of amphoteric surfactants and the correlation between the determination methods applied" Comunicaciones presentadas a las Jornadas del Comité Español de la Detergencia. (1997), 27, 241-255) ^{b}[J.Soc.Cosmet.Chem. 32, 271-286, (July-August 1989)] (20 wt.-% olive oil + 15 wt.-% oleic acid + 15 wt.-% Coconut acid + 15 wt.-% Myristyl Myristate + 10 wt.-% palmitic acid + 10 wt.-% paraffin waxes + 5 wt.-% stearic acid + 5 wt.-% Squalane + 5 wt.-% Cholesterol) | | | | |

### 4. Foam density

Aqueous surfactant solutions (15 wt.% a.m.) containing sodium laureth-2 sulfate and different compounds in a ratio 3:1 (based on active matter) were prepared. The foaming power of 1 wt.-% active matter surfactant solutions (pH 6-7; 25°C; 15°dH) were prepared and its foam density was determined according to the Bubbling method (X. Domingo, L. Fiquet, H. Meijer "Foam Ability/Stability of Surfactants" Tenside Surf. Det.29 (1992) 1,pp. 16-22). This parameter can be correlated to the ability of surfactant solutions to form close, dense lathers.

The foam density is measured after 30 seconds in duplicate. The values shown in Table 3 are the average of the obtained results.

**Table 3 - Foam Density Values**

| | Surfactant compositions | Mixture SLES/Foam booster 3:1 | | Foam density (g/L) |
|---|---|---|---|---|
| Invention | S1' | SLES | Aqueous compos. 1 | 7.1 |
| Comparative Experiments | SC1' | SLES | C1 | 5.2 |
| | SC3' | SLES | C3 | 5.3 |

From the experimental results, it can be concluded that the compositions according to the invention not only are pumpable even at high concentration, but also show unexpected foam properties if compared with the foam behaviour of glycerol and alkyl ether carboxylates alone.

Furthermore, it can be observed that although known alcohols (e.g. ethanol) can be used to obtain relatively pumpable highly concentrated aqueous solutions of alkyl ether carboxylates, the foam properties of the resultant compositions are poor.

### 5. Cosmetic compositions (weight percentages with respect to the composition)

| Example No | 5.1 | 5.2 | 5.3 | 5.4 | 5.5 |
|---|---|---|---|---|---|
| Sodium Laureth-2 Sulfate | 8 | 13 | 11 | 12 | 5 |
| Cocamidopropylbetaine | 4 | 0.5 | | 5 | 7 |
| Sodium Cocoylglutamate | | | | | 4 |
| Sodium Cocoamphoacetate | | | | 1 | |
| Decyl Glucoside | 2 | | 2 | | |
| Foam Booster³ | 0.7 | 0.5 | 3 | 1.5 | 7 |
| Glycol Distearate | | 1 | | 1 | |
| Acrylate Copolymer | 0.5 | 3 | 0.8 | | 0.5 |
| Styrene/Acrylate Copolymer | | | | 0.2 | |
| Polyquaternium-10 | 0.2 | | 0.2 | 0.2 | 0.2 |
| Hydroxypropyl Guar Hydroxypropyl Trimonium Chloride | 0.2 | 0.2 | | 0.1 | 0.1 |
| PEG-6 Caprylic/Capric Glycerides | | | | | 0.5 |
| PEG-40 Hydrogenated Castor Oil | 0.8 | 1 | | 0.2 | 0.5 |
| Glycereth-2 Cocoate | 2 | | | | |
| Paraffin Oil | | | 25 | | 0.8 |
| Soya Oil | | | 25 | | |
| Sodium benzoate | 0.45 | | 0.3' | 0.4 | |
| Sodium Salicilate | 0.2 | | 0.2 | 0.4 | |
| Citric Acid | 0.5 | 0.5 | 0.5 | 0.5 | 0.5 |
| Perfume | s.q. | s.q. | s.q. | s.q. | s.q. |
| Water | Ad 100 | Ad 100 | Ad 100 | Ad 100 | Ad 100 |

| | | | | | |
|---|---|---|---|---|---|
| ³Foam Booster: Aqueous composition 1 | | | | | |

## Claims

1. An aqueous composition with a water content of at most 55 wt.% water, preferably at most 45 wt.%, based on the total weight of the composition, comprising:
(a) one or more alkyl ether carboxylates of the following formula I
R-O-(CH₂CH₂O)ₙCH₂COO⁻ M⁺ Formula I
wherein
R is an alkyl residue having 6 to 22 carbon atoms;
n has a value in the range of 2.0 to 4.5;
and M⁺ is an appropriate cation, selected from the group consisting of an alkali metal, an alkaline earth metal, ammonium, an alkylammonium, an alkanolammonium or a glucammonium;
**characterized in that** the composition contains
(b) glycerol in an amount of 0.5 to 15 wt.% based on the total weight of the composition.

2. The composition according to claim 1, **characterized in that** the weight ratio of component (a) to component (b) is in the range of 3:1 to 9:1, preferably in the range from 4:1 to 7:1.

3. The composition according to claim 1 or 2, **characterized in that** the total amount of component (b) is in the range of 0.5 to 15 wt.%, preferably 3 to 9 wt.% based on the total weight of the composition.

4. The composition according to any one of claims 1 to 3, **characterized in that** in the compound of formula I R is an alkyl residue having 12 to 14 carbon atoms, n has a value in the range of 2.5 to 4.0 and M⁺ is sodium or potassium.

5. The composition according to claim 4, **characterized in that** in the compound of formula I n is 3.0.

6. The composition according to claim 4, **characterized in that** in the compound of formula I n is 4.0.

7. The composition according to any one of claims 1 to 6, further comprising component (c), being an ethoxylated fatty alcohol of formula II
R-O-(CH₂CH₂O)ₙ-H Formula II
wherein R and n have the same meaning as for formula I.

8. The composition according to claim 7, wherein the mole ratio of alkyl ether carboxylate (a) to ethoxylated fatty alcohol (c) is in the range of 3:7 to 7.5:2.5.

9. The composition according to anyone of claims 1 to 8 comprising, based on the total weight of the composition,
(i) from 20 to 45 wt.% of component (a)
(ii) from 3 to 9 wt.% of component (b)
(iii) from 20 to 45 wt.% of component (c)

10. A process for the preparation of aqueous compositions according to claims 1 to 9, wherein fatty alcohols are ethoxylated and subsequently carboxymethylated to obtain alkyl ether carboxylates of the following formula I
R-O-(CH₂CH₂O)ₙ-CH₂COO⁻ M⁺ Formula I
wherein R, n and M⁺ have the same meaning as above,
**characterized in that** glycerol is added in an amount of 0.5 to 15 wt.% to the crude alkyl ether carboxylates and finally the pH is adjusted to 5.5-6.5.

11. A process for the preparation of aqueous compositions according to claims 1 to 9, wherein fatty alcohols are ethoxylated and subsequently carboxymethylated to obtain alkyl ether carboxylates of the following formula I
R-O-(CH₂CH₂O)ₙ-CH₂COO⁻ M⁺ Formula I
wherein R, n and M⁺ have the same meaning as above,
**characterized in that**
i) the crude alkyl ether carboxylates are converted into alkyl ether carboxylic acids and purified;
ii) glycerol is added in an amount of 0.5 to 15 wt.% to the purified alkyl ether carboxylic acids; and
iii) the pH is adjusted to 5.5-6.5.

12. The process according to claims 10 or 11, **characterized in that** the weight ratio of alkyl ether carboxylate (component a) and ethoxylated fatty alcohol (component c) to glycerol (component b) is in the range of 5:1 to 15:1, preferably in the range from 8:1 to 13:1.

13. A use of an aqueous composition as defined in any of claims 1 to 9 as foam-enhancing agent or foam booster for anionic, cationic, non-ionic and/or amphoteric surfactants or mixtures thereof.

14. A cosmetic composition comprising the aqueous composition of any one of claims 1 to 9 and one or more surfactants, selected from anionic surfactants, non-ionic surfactants, cationic surfactants, and/or amphoteric surfactants, wherein the alkyl ether carboxylate (a) and glycerol (b), and optionally ethoxylated fatty alcohol (c), are present in a total amount of 0.5 to 25 wt.%, preferably 1 to 20 wt.-%, most preferably 2 to 10 wt.-%, with respect to the total weight of the cosmetic composition.

15. The cosmetic compositions according to claim 14, **characterized in that** they are liquid soaps, hair shampoos, body shampoos, hair lotions, foam baths, shower baths, creams, gels, lotions, shaving preparations, after-shaving preparations, alcoholic and aqueous/alcoholic solutions, or emulsions.

16. The cosmetic compositions according to claim 14 or 15, **characterized in that** the total amount of surfactants (active matter), including components (a) and (c), if present, is in the range of 10 to 35 wt.-% with respect to the total weight of the composition.

17. The cosmetic composition according to any one of claims 14 to 16, containing an anionic surfactant such that the weight ratio of anionic surfactant to the total weight of components (a), (b) and, if present, (c), is in the range of 2:1 to 6:1, preferably 3:1 to 5:1.

18. The cosmetic compositions according to any one of claims 14 to 17, wherein the anionic surfactant is sodium lauryl ether sulfate.

## Patentansprüche

1. Wäßrige Zusammensetzung mit einem Wassergehalt von höchstens 55 Gew.% Wasser, vorzugsweise höchstens 45 Gew.% Wasser, bezogen auf das Gesamtgewicht der Zusammensetzung, umfassend:
(a) ein oder mehrere Alkylethercarboxylate der folgenden Formel (I)
R-O-(CH₂CH₂O)ₙ-CH₂COO⁻ M⁺ Formel (I)
worin
R ein Alkyl-Rest mit 6 bis 22 Kohlenstoffatomen ist;
n einen Wert im Bereich von 2,0 bis 4,5 aufweist;
und M⁺ ein geeignetes Kation ist, das aus der Gruppe ausgewählt wird, die aus einem Alkalimetall, einem Erdalkalimetall, Ammonium, einem Alkylammonium, einem Alkanolammonium oder einem Glucammonium besteht;
**dadurch gekennzeichnet, daß** die Zusammensetzung
(b) Glycerin in einer Menge von 0,5 bis 15 Gew.%, bezogen auf das Gesamtgewicht der Zusammensetzung enthält.

2. Zusammensetzung gemäß Anspruch 1, **dadurch gekennzeichnet, daß** das Gewichtsverhältnis der Komponente (a) zu der Komponente (b) im Bereich von 3:1 bis 9:1, vorzugsweise im Bereich 4:1 bis 7:1 liegt.

3. Zusammensetzung gemäß Anspruch 1 oder 2, **dadurch gekennzeichnet, daß** die Gesamtmenge der Komponente (b) im Bereich von 0,5 bis 15 Gew.%, vorzugsweise 3 bis 9 Gew.%, bezogen auf das Gesamtgewicht der Zusammensetzung liegt.

4. Zusammensetzung gemäß mindestens einem der Ansprüche 1 bis 3, **dadurch gekennzeichnet, daß** in der Verbindung der Formel (I) R ein Alkyl-Rest mit 12 bis 14 Kohlenstoffatomen ist, n einen Wert im Bereich von 2,5 bis 4,0 aufweist und M⁺ Natrium oder Kalium ist.

5. Zusammensetzung gemäß Anspruch 4, **dadurch gekennzeichnet, daß** in der Verbindung der Formel (I) n 3,0 ist.

6. Zusammensetzung gemäß Anspruch 4, **dadurch gekennzeichnet, daß** in der Verbindung der Formel (I) n 4,0 ist.

7. Zusammensetzung gemäß mindestens einem der Ansprüche 1 bis 6, weiterhin umfassend Komponente (c), die ein ethoxylierter Fettalkohol der Formel (II) ist
R-O-(CH₂CH₂O)ₙ-H ) Formel (II
worin R und n dieselbe Bedeutung wie für Formel (I) haben.

8. Zusammensetzung gemäß Anspruch 7, worin das Molverhältnis des Alkylethercarboxlates (a) zu dem ethoxylierten Fettalkohol (c) im Bereich von 3:7 bis 7,5:2,5 liegt.

9. Zusammensetzung gemäß mindestens einem der Ansprüche 1 bis 8, umfassend, bezogen auf das Gesamtgewicht der Zusammensetzung,
(i) 20 bis 45 Gew.% der Komponente (a)
(ii) 3 bis 9 Gew.% der Komponente (b)
(iii) 20 bis 45 Gew.% der Komponente (c).

10. Verfahren zur Herstellung der wäßrigen Zusammensetzungen gemäß den Ansprüchen 1 bis 9, worin Fettalkohole ethoxyliert und anschließend unter Erhalt von Alkylethercarboxylaten der folgenden Formel (I) carboxymethyliert werden
R-O-(CH₂CH₂O)ₙ-CH₂COO⁻ M⁺ Formel (I)
worin R, n und M⁺ dieselbe Bedeutung wie oben haben,
**dadurch gekennzeichnet, daß** Glycerin in einer Menge von 0,5 bis 15 Gew.% zu den rohen Alkylethercarboxylaten hinzugefügt wird und schließlich der pH-Wert auf 5,5-6,5 eingestellt wird.

11. Verfahren zur Herstellung von wäßrigen Zusammensetzungen gemäß den Ansprüchen 1 bis 9, worin Fettalkohole ethoxyliert und anschließend unter Erhalt von Alkylethercarboxylaten der folgenden Formel (I) carboxymethyliert werden
R-O-(CH₂CH₂O)ₙ-CH₂COO⁻ M⁺ Formel (I)
worin R, n und M⁺ dieselbe Bedeutung wie oben haben,
**dadurch gekennzeichnet, daß**
i) die rohen Alkylethercarboxylate zu Alkylethercarbonsäuren umgesetzt und gereinigt werden;
ii) Glycerin in einer Menge von 0,5 bis 15 Gew.% zu den gereinigten Alkylethercarbonsäuren hinzugefügt wird und
iii) der pH-Wert auf 5,5-6,5 eingestellt wird.

12. Verfahren gemäß den Ansprüchen 10 oder 11, **dadurch gekennzeichnet, daß** das Gewichtsverhältnis des Alkylethercarboxylats (Komponente a) und des ethoxylierten Fettalkohols (Komponente c) zu Glycerin (Komponente b) im Bereich von 5:1 bis 15:1, vorzugsweise im Bereich von 8:1 bis 13:1, liegt.

13. Verwendung einer wäßrigen Zusammensetzung, wie in mindestens einem der Ansprüche 1 bis 9 definiert, als Schaumverbesserungsmittel oder Schaumverstärker für anionische, kationische, nicht-ionische und/oder amphotere Tenside oder Mischungen davon.

14. kosmetische Zusammensetzung, umfassend die wäßrige Zusammensetzung gemäß mindestens einem der Ansprüche 1 bis 9 und ein oder mehrere Tenside, ausgewählt aus anionischen Tensiden, nicht-ionischen Tensiden, kationischen Tensiden und/oder amphoteren Tensiden, wobei das Alkylethercarboxylat (a) und Glycerin (b) und gegebenenfalls der ethoxylierte Fettalkohol (c) in einer Gesamtmenge von 0,5 bis 25 Gew.%, vorzugsweise 1 bis 20 Gew.%, am meisten bevorzugt 2 bis 10 Gew.%, in bezug auf das Gesamtgewicht der kosmetischen Zusammensetzung vorhanden sind.

15. Kosmetische Zusammensetzungen gemäß Anspruch 14, **dadurch gekennzeichnet, daß** sie flüssige Seifen, Haarshampoos, Körpershampoos, Haarlotionen, Schaumbäder, Duschbäder, Cremes, Gels, Lotionen, Rasierzubereitungen, Aftershave-Zubereitungen, alkoholische und wäßrige/alkoholische Lösungen oder Emulsionen sind.

16. Kosmetische Zusammensetzungen gemäß Anspruch 14 oder 15, **dadurch gekennzeichnet, daß** die Gesamtmenge an Tensiden (aktive Substanz), einschließlich der Komponenten (a) und (c), falls vorhanden, im Bereich von 10 bis 35 Gew.% in bezug auf das Gesamtgewicht der Zusammensetzung liegt.

17. Kosmetische Zusammensetzungen gemäß mindestens einem der Ansprüche 14 bis 16, die ein anionisches Tensid enthält, so daß das Gewichtsverhältnis des anionischen Tensids zu der Gesamtmenge der Komponenten (a), (b) und, falls vorhanden, (c) im Bereich von 2:1 bis 6:1, vorzugsweise 3:1 bis 5:1, liegt.

18. Kosmetische Zusammensetzungen gemäß mindestens einem der Ansprüche 14 bis 17, worin das anionische Tensid Natriumlaurylethersulfat ist.

## Revendications

1. Composition aqueuse avec une teneur en eau d'au plus 55% en poids d'eau, préférablement au plus 45% en poids, sur la base du poids total de la composition, comprenant :
(a) un ou plusieurs carboxylate(s) d'éther alkylique de formule I suivante
R-O- (CH₂CH₂O)ₙ-CH₂COO⁻ M⁺ Formule I
dans laquelle
R est un résidu alkyle ayant 6 à 22 atomes de carbone ;
n a une valeur dans la plage de 2,0 à 4,5 :
et M⁺ est un cation approprié, choisi parmi le groupe consistant en un métal alcalin, un métal alcalinoterreux, l'ammonium, un alkylammonium, un alcanolammonium ou un glucammonium ;
**caractérisée en ce que** la composition contient
(b) du glycérol dans une quantité de 0,5 à 15% en poids sur la base du poids total de la composition.

2. Composition selon la revendication 1, **caractérisée en ce que** le rapport en poids du composant (a) sur le composant (b) est dans la plage de 3:1 à 9:1, préférablement dans la plage de 4:1 à 7:1.

3. Composition selon la revendication 1 ou 2, **caractérisée en ce que** la quantité totale du composant (b) est dans la plage de 0,5 à 15% en poids, préférablement 3 à 9% en poids sur la base du poids total de la composition.

4. Composition selon l'une quelconque des revendications 1 à 3, **caractérisée en ce que** dans le composé de Formule I, R est un résidu alkyle ayant 12 à 14 atomes de carbone, n a une valeur dans la plage de 2,5 à 4,0 et M⁺ est du sodium ou du potassium.

5. Composition selon la revendication 4, **caractérisée en ce que** dans le composé de Formule I, n est 3,0.

6. Composition selon la revendication 4, **caractérisée en ce que** dans le composé de Formule I, n est 4,0.

7. Composition selon l'une quelconque des revendications 1 à 6, comprenant en outre un composant (c), étant un alcool gras éthoxylé de formule II
R-O-(CH₂CH₂O)ₙ-H Formule II
dans laquelle R et n ont la même signification que pour la formule I.

8. Composition selon la revendication 7, dans laquelle le rapport en moles du carboxylate d'éther alkylique (a) sur l'alcool gras éthoxylé (c) est dans la plage de 3:7 à 7,5:2,5.

9. Composition selon l'une quelconque des revendications 1 à 8, comprenant, sur la base du poids total de la composition,
(i) de 20 à 45% en poids du composant (a)
(ii) de 3 à 9% en poids du composant (b)
(iii) de 20 à 45% en poids du composant (c).

10. Procédé pour la préparation de compositions aqueuses selon les revendications 1 à 9, dans lequel des alcools gras sont éthoxylés et subséquemment carboxyméthylés pour obtenir des carboxylates d'éther alkylique de formule I suivante
R-O-(CH₂CH₂O)ₙ-CH₂COO⁻ M⁺ Formule I
dans laquelle
R, n et M⁺ ont le même sens que ci-dessus,
**caractérisé en ce que** du glycérol est ajouté dans une quantité de 0,5 à 15% en poids aux carboxylates d'éther alkylique bruts et enfin le pH est ajusté à 5,5-6,5.

11. Procédé pour la préparation de compositions aqueuses selon les revendications 1 à 9, dans lequel des alcools gras sont éthoxylés et subséquemment carboxyméthylés pour obtenir des carboxylates d'éther alkylique de formule I suivante
R-O-(CH₂CH₂O)ₙ-CH₂COO⁻ M⁺ Formule I
dans laquelle
R, n et M⁺ ont le même sens que ci-dessus,
**caractérisé en ce que**
i) les carboxylates d'éther alkylique bruts sont convertis en acides carboxyliques d'éther alkylique et purifiés ;
ii) du glycérol est ajouté dans une quantité de 0,5 à 15% en poids aux acides carboxyliques d'éther alkylique purifiés ; et
iii) le pH est ajusté à 5,5-6,5.

12. Procédé selon les revendications 10 ou 11, **caractérisé en ce que** le rapport en poids du carboxylate d'éther alkylique (composant a) et de l'acide gras éthoxylé (composant c) sur le glycérol (composant b) est dans la plage de 5:1 à 15:1, préférablement dans la plage de 8:1 à 13:1.

13. Utilisation d'une composition aqueuse telle que définie dans l'une quelconque des revendications 1 à 9 comme agent d'amélioration de mousse ou renforçateur de mousse pour des agents tensioactifs anioniques, cationiques, non ioniques et/ou amphotères ou des mélanges de ceux-ci.

14. Composition cosmétique comprenant la composition aqueuse selon l'une quelconque des revendications 1 à 9 et un ou plusieurs agent(s) tensioactif(s), choisi(s) parmi des agents tensioactifs anioniques, des agents tensioactifs non ioniques, des agents tensioactifs cationiques, et/ou des agents tensioactifs amphotères, dans laquelle le carboxylate d'éther alkylique (a) et le glycérol (b), et facultativement l'acide gras éthoxylé (c), sont présents dans une quantité totale de 0,5 à 25% en poids, préférablement 1 à 20% en poids, le plus préférablement 2 à 10% en poids, par rapport au poids total de la composition cosmétique.

15. Compositions cosmétiques selon la revendication 14, **caractérisées en ce qu'**elles sont des savons liquides, des shampooings pour les cheveux, des shampooings pour le corps, des lotions capillaires, des bains moussants, des gels douche, des crèmes, des gels, des lotions, des préparations pour le rasage, des préparations pour l'après-rasage, des solutions alcooliques et aqueuses/alcooliques, ou des émulsions.

16. Compositions cosmétiques selon la revendication 14 ou 15, **caractérisées en ce que** la quantité totale d'agents tensioactifs (matière active), incluant les composants (a) et (c), s'il est présent, est dans la plage de 10 à 35% en poids par rapport au poids total de la composition.

17. Composition cosmétique selon l'une quelconque des revendications 14 à 16, contenant un agent tensioactif anionique de telle sorte que le rapport en poids de l'agent tensioactif anionique sur le poids total des composants (a), (b) et, s'il est présent, (c), est dans la plage de 2:1 à 6:1, préférablement 3:1 à 5:1.

18. Compositions cosmétiques selon l'une quelconque des revendications 14 à 17, dans lesquelles l'agent tensioactif anionique est le lauryléthersulfate de sodium.
